# EUROPEAN PATENT SPECIFICATION

(11) **EP 1 393 049 B1**
(45) Date of publication and mention of the grant of the patent: **25.07.2007**
(21) Application number: 02732920.0
(22) Date of filing: 06.06.2002
(51) Int. Cl.: G01N 21/76

(54) **TOXICITY MONITOR**
TOXIZITÄTSMONITOR
DISPOSITIF DE MESURE DE LA TOXICITE

(30) Priority: 06.06.2001 GB 0113793
(43) Date of publication of application: 03.03.2004
(73) Proprietor: The University of Surrey, Guildford, Surrey GU2 7XH (GB)
(72) Inventor: HART, Timothy David, Bicester, Oxfordshire OX26 6YH (GB); BAINTON, Nigel John, Worcester Park, Surrey KT4 7AL (GB)
(74) Representative: Butler, Lance
(86) International application number: PCT/GB2002/002594
(87) International publication number: WO 2002/099121

(56) References cited:
- EP-A- 0 757 244
- US-A- 4 772 453
- US-A- 5 919 645
- CHUN U ET AL: "Continuous pollution monitoring using Photobacterium phosphoreum" RESOURCES CONSERVATION AND RECYCLING, ELSEVIER SCIENCE PUBLISHER, AMSTERDAM, NL, vol. 18, no. 1, 1 November 1996 (1996-11-01), pages 25-40, XP004016613 ISSN: 0921-3449
- BULICH A: "Mutatox: a genotoxicity assay using luminescent bacteria." SCHRIFTENREIHE DES VEREINS FUR WASSER-, BODEN- UND LUFTHYGIENE. GERMANY 1992, vol. 89, 1992, pages 763-770, XP009014169 ISSN: 0300-8665

## Description

This invention relates to a method and an instrument for measuring toxicity, with particular reference to measuring the toxicity of solids of liquids, for example soil or water samples.

Increasing demand for redevelopment of so-called "brownfield" sites, i.e. disused sites previously employed for other purposes, increasingly requires checks to be made for whether the activities of previous occupants of the site have left any toxic residues in solid or liquid phases within its soil or strata. In preparing and redeveloping the site the developer must ensure the health and safety of site workers and visitors during preparation and construction phases and must also have regard to the health and safety of members of the public who will subsequently visit or use the finished project. The need for checks is especially important for projects including new agricultural use or including domestic gardens, given that subsequent use of contaminated soil to grow crops could result in toxic material entering the food chain.

A wide variety of methods and systems for toxicity testing have been proposed and used for many years, although they have largely been concerned with detection of toxic contaminants in air or water rather than in soil or solids material or ground water or leachate. The present invention relates to a detection method and system for use with solids or liquids, e.g. soil, and is based on the measurement of changes in bacterial bioluminescence caused by exposure of the luminescent bacterial micro-organisms to a toxic material in an observed cell, where light production is directly correlated to the metabolic health of the cell. Because the light-generating activity of the micro-organisms is adversely affected by the toxic material their luminescence provides a measure of the degree of contamination.

US Patent No. 3,370,175 provides an early example of the use of luminescent micro-organisms for detection of toxic substances in air. Sensors are employed to detect changes in the light output from the micro-organisms and thus to indicate the presence and relative concentration of the toxic materials.

GB Patent No. 2,005,018 describes a method in which a material suspected of containing a toxic substance is intermixed with an aqueous suspension of luminous micro-organisms of known light output, and the output of light from the luminous micro-organisms sensed after contact with the material is compared with a base line output of the luminous micro-organisms before the intermixing.

More recent disclosures relate to specific configurations of detection units or of the procedures used in them. US Patent No. 5,082,628 relates to a laboratory instrument comprising multiple wells to receive samples of material to be tested. A light detector is located beneath the wells, which have transparent bases, and the wells are passed in turn into alignment with the detector. US Patent No. 5,919,645 describes a method for the direct determination of the toxicity of a solid material by direct contact with a luminous micro-organism.

The present invention is concerned with the provision of a portable robust, reliable detection method and unit suitable for measuring the generic toxicity of solids or liquids, for example soil, samples in the field.

According to a first aspect of the present invention there is provided a device for measuring the generic toxicity of solids or liquids which comprises a container to hold bioluminescent bacteria and to receive pollutants separated from the solids or liquids, and an opto-electronic unit to detect the level of bioluminscence from the container, characterised in that the container for bacteria and pollutants comprises one or more modular inspection trays each containing an array of cells which each have a transparent or translucent base, and the opto-electronic unit includes an array of light sensors arranged in a configuration complementary to that of the inspection trays.

According to a second aspect of the invention there is provided a device for measuring the generic toxicity of solids or liquid samples which comprises extraction means to separate pollutants from the liquids or solids, a container to hold bioluminescent bacteria and to receive pollutants separated from the solids or liquids, and an opto-electronic unit to detect the level of bioluminescence from the container, characterised in that the extraction means comprises one or more modular feed trays each containing an array of cells which each have a porous base, the container for bacteria and pollutants comprises one or more modular inspection trays each containing an array of cells which each have a transparent or translucent base and which are arranged in a configuration complementary to that of the feed trays, and the opto-electronic unit includes an array of light sensors arranged in a configuration complementary to those of the feed and inspection trays.

A third aspect of the invention provides a method of measuring the generic toxicity of solids or liquids samples in which pollutants are first separated from the solids or liquids, are then brought into contact with bioluminscent bacteria and the level of bioluminescence is then detected and monitored, characterised in that the pollutants are passed into a modular inspection tray containing an array of cells containing bioluminescent bacteria which each have a transparent or translucent base, and the level of luminscence from the inspection cells is detected by an array of light sensors arranged in a configuration complementary to that of the inspection tray.

A fourth aspect of the invention further provides a method for measuring the generic toxicity of solids or liquids samples in which pollutants are first separated from the solids or liquids, are then brought into contact with bioluminescent bacteria and the level of bioluminescence is then detected and monitored, characterised in that the solids or liquids samples are placed together with a pollutant-extracting liquid into one or more modular feed trays each containing an array of cells which each have a porous base, extracted pollutants pass through the porous bases into a modular inspection tray containing an array of cells containing bioluminescent material which each have a transparent or translucent base and which are arranged in a configuration complementary to that of the feed trays, and the level of luminescence from the inspection cells is detected by an array of light sensors arranged in a configuration complementary to those of the feed and inspection trays.

The invention is advantageous in providing a rapid on-site toxicity audit system (ROTAS^{™}). A particular advantage of the invention is that the system can be produced in a portable form, permitting ready on-site use. The system provides a simple one-step extraction process to facilitate use by unskilled operators in the field. It can be used as a tool for toxicity audit, site investigation, monitoring and validation of remediation processes or activities, and routine safety assessment.

The toxins detectable by the system are necessarily those that are extractable from the soil, from which they can be introduced into a biological cell. The extractable toxins, otherwise known as mobile or bioavailable toxins, are not firmly bound to the soil minerals and can therefore be leached therefrom or extracted with particular solvents, especially in the aqueous phase.

A further advantage of the invention is that the multi-cellular configuration of the trays enables simultaneous analysis of multiple samples, some of which can beneficially be control samples of known properties so as to permit accurate comparisons with control values, for example of zero pollution or of permissible threshold levels of pollution.

The feed trays can be used for the supply to the inspection trays of either soil extracts for testing or activating agents used in preparing bacteria for use. The peripheral lower edge of the feed trays and the peripheral upper edge of the inspection trays preferably have complementary tongue and groove configurations so that when the trays are brought together for transfer of material from the upper (feed) tray to the lower (inspection) tray a seal is formed between the respective peripheries. The tongue and groove configurations are preferably asymmetrical so that the trays can only be fitted together in one direction, thereby avoiding confusion over the cell contents.

The feed and inspection trays are preferably constructed of moulded plastic material, which may contain an additive such as Teflon^{®} or PTFE to prevent the sorption of pollutants to the cell walls. The base of each of the cells in the feed trays is preferably constructed of porous plastic so as to permit transfer of material from the cells. The base of each of the cells in the inspection trays is preferably constructed of optical-grade transparent plastic to provide for inspection of light activity within the cells.

The feed trays and the inspection trays preferably include sealable but detachable lids. A sealed lid allows for a tray to be pre-filled with required materials, for example extractant fluid for solids or liquids samples or activating agent for bacteria.

The lids employed for the feed trays preferably include a shaped orifice to receive a pressurising syringe to create an excess pressure in the feed trays to assist transfer of material to the inspection trays.

The opto-electrical unit according to the invention should include a measurement zone to receive an inspection tray containing bacteria and solids or liquids extracts.

In one convenient embodiment of the invention the opto-electrical unit further includes an incubation zone to store one or more inspection trays for activation of the bacteria prior to addition of solids or liquids extracts. The incubation zone preferably includes a thermo control element to regulate the zone to give an optimum level of heating for the incubation.

The opto-electrical unit is preferably battery powered so as to facilitate on-site working when no mains power is available. The battery is preferably of a rechargeable type.

Light sensors, for example photodiodes, need to be located at the base of the measurement zone, preferably at least one sensor per inspection cell, to receive bioluminescence from the inspection cells. Each sensor may have an associated lens to focus the bioluminescence from each of the cells.

The opto-electrical unit preferably includes a micro-controller to mediate data acquisition and storage. It may be desirable and convenient to employ programmed algorithms to assist its data analysis. A typical output display for each dataset is a two-dimensional representation of the inspection tray, with toxicity values expressed as percentage in bioluminescence with respect to control values. The micro-controller can be linked to another electronic device for further processing of the received data.

Various bioluminescent bacteria can be employed for the toxicity measurement. *Vibrio fischeri* has proved to be particularly suitable but other bacteria to use include *Vibrio harveyi, Photorhabdus luminescens, Photobacterium phosphoreum,* or any genetically engineered bacterium, a micro-organism or cell(s) which displays luminescence. The bacteria can be stored in freeze-dried form, reactivated when required, for example by addition of an activation medium, and incubated at appropriate temperatures (for example 15°C - 37°C) for a sufficient period (typically about 60 minutes) until they reach a sufficient level of bioluminescent activity. A defined volume of the solid or liquid extract to be tested is then added to the activated bacteria.

Suitable activation media, and typical concentrations in which they can be employed, include by way of example sodium chloride (1-4% w/v pH (5-8) buffer).

For certain pollutants, for example benzene, it may be advantageous to include in the activation medium a substance that increases the sensitivity of the bacteria to that pollutant. The substance may be surfactant-based or be any other substance capable of increasing the sensitivity of the inspection cells to particular pollutants. The soil samples for analysis will usually be collected by hand. The soil should preferably be crushed and/or sieved to a particle size sufficiently small (for example less than 50 µm) to facilitate extraction of toxic residues, and homogenised to give samples of a relatively uniform composition. Suitable extractant liquids include solvents such as methanol or acetone, surfactants, solutions based on ethylenediaminetetraacetic acid (EDTA), salts such as ammonia acetate, and mixtures thereof.

Agitation of the cell contents is desirable at various stages of the testing procedure to ensure thorough mixing of the materials in the cell. The agitation can be facilitated by placing over the respective tray a lid which seals each of the individual cells and thus allows the tray as a whole to be shaken or vibrated for a required period without loss of material from any of the cells.

For control purposes some of the cells in the inspection trays may comprise the following standard contents:
a cell with no soil extract, producing 100% bioluminescence (positive control).
a cell with a standard toxin, for example phenol, zinc sulphate, sodium dodecyl sulphate (SDS) or lysozyme, to reduce bioluminescence by 100% (negative control).
cells with standard toxins corresponding to pollutant trigger levels relevant to the site under investigation and the risk assessment system followed.

Possible standard toxins to include are benzene, toluene, ethylbenzene and xylene (BTEX), representative of aromatics; benzopyrene, representative of polyaromatic hydrocarbons; aroclors (commercial mixtures of polychlorinated biphenols); and heavy metals such as copper, lead, mercury or cadmium.

The invention is further described, by way of non-limiting example, with reference to the accompanying figures, in which:
Figure 1 shows an end view of modular cellular feed trays and inspection trays according to the invention, and
Figure 2 shows a perspective view of a detection unit according to the invention, including a slightly different version of modular cellular inspection trays according to the invention.
Figure 3 is a chart showing the relationship of various electronic components to each other in an opto-electronics unit according to the invention.

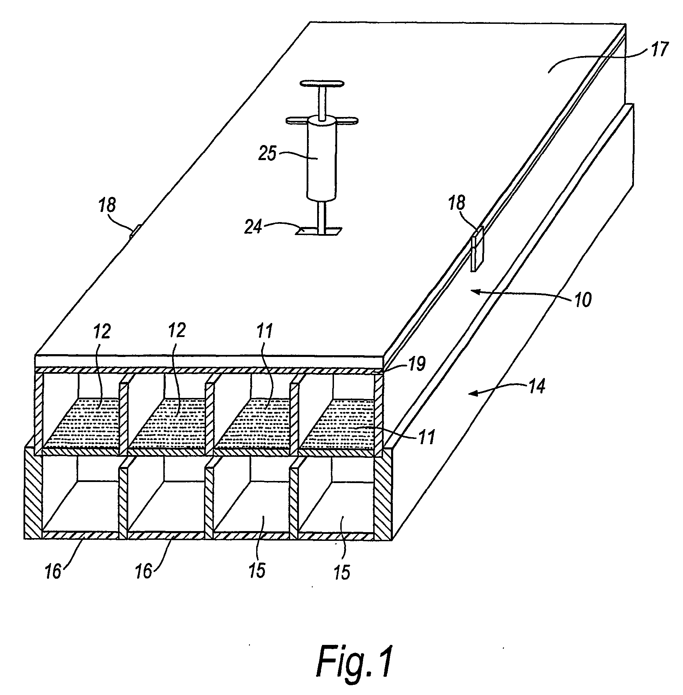

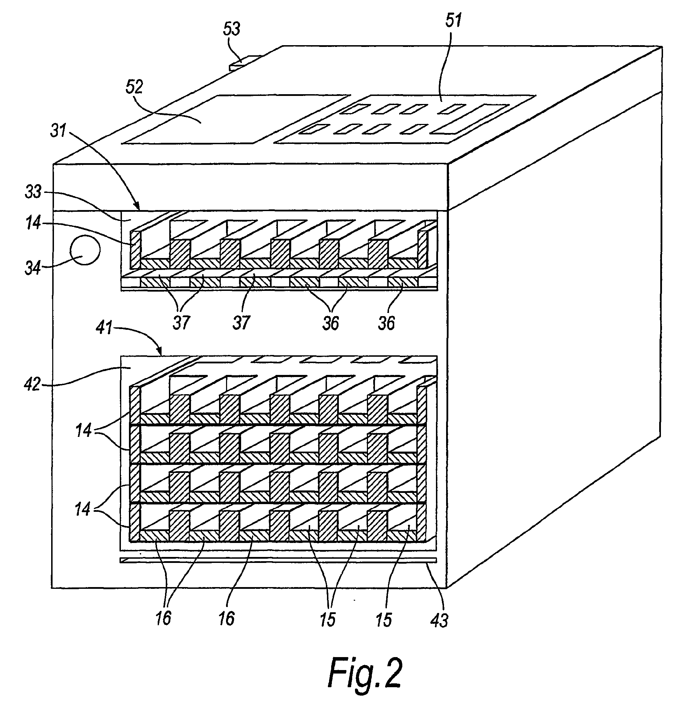

In Figure 1 the visible sides of the trays and in Figure 2 the visible fronts of the trays are shown in section so as to indicate their internal configuration.

Figure 1 shows a feed tray 10 mounted above an inspection tray 14. The peripheral lower edge of tray 10 and the peripheral upper edge of tray 14 have complementary tongue and groove configurations so that when the trays are brought together as shown in Figure 1 the upper tray 10 fits snugly into the lower tray 14 to form a seal between the respective peripheries. The tongue and groove configurations are asymmetrical so that the trays 10 and 14 can only be fitted together in one direction.

The tray 10 has multiple internal cells 11 each with a porous base 12 to provide for feed of material to tray 14. The tray 14 has multiple internal cells 15 each with a base 16 of optical-grade transparent plastic to provide for inspection of light activity within the cells 15. The illustrated trays 10 and 14 each have 24 cells, arranged in four rows of six and of complementary configuration and shape to each other so that the inspection cells 15 are aligned beneath the corresponding feed cells 11 when the trays 10 and 14 are fitted together.

Internal walls of the cells 11 and 15 are of lesser height than the side walls of the respective trays 10 and 14 to allow for a uniform vapour pressure in the head space above the cells in the respective trays.

Figure 2 shows an opto-electrical unit according to the invention and containing several inspection trays 14. It has a casing 30 made from heat-, water- and solvent-resistant insulating plastic to provide a durable covering and minimise heat loss. The unit includes two tray-receiving zones, an upper zone 31 and a lower zone 41. The upper zone 31 is a measurement zone to receive an inspection tray 14 containing bacteria and solids or liquid extracts, for example soil extracts. The lower zone 41 is an incubation zone to store several inspection trays 14 at optimum temperature for activation of the bacteria prior to addition of the extracts.

The opto-electrical unit is powered by a rechargeable battery, capable of being used or charged from a 12 V car cigar lighter socket or transformer linked to a 240 V mains supply.

The measurement zone 31 has an opaque hinged flap 33 (shown as transparent in Figure 2 so as to indicate its contents) which is opened by an eject button 34 to permit an inspection tray 14 to be inserted and locked in position. The flap 33 is then firmly closed so as prevent the entry of any extraneous light source into the zone 31. The eject button 34 allows release and removal of the tray after measurement.

An array of 24 light sensors 36 (photodiodes) is located at the base of the measurement zone 31, with each sensor being positioned to be in alignment with a cell 15 in an inserted inspection tray 14. The array of light sensors 36 is fitted with a corresponding array of lenses 37 to focus bioluminescence from each of the cells 15 to its respective sensor 36. In an alternative embodiment the lenses may be omitted.

The incubation zone 41 has a hinged closure flap 42. It further includes a thermo control, for example a heating pad 43 located in the base so as to create and sustain the optimum incubation temperature, e.g. 15°C to 37°C.

The upper part of the opto-electrical unit, above the measurement zone 31, houses a micro-controller which includes a soft-touch control keypad 51 and an LCD display screen 52 as a graphical interface for user interaction. The micro-controller mediates data acquisition and storage, and performs data analysis with the aid of programmed algorithms. The LCD display allows datasets to be displayed in the field and operation of the unit to be customised via a series of menus, accessible by the keypad 51. A typical output display for each dataset is a two-dimensional representation of the inspection tray 14 with toxicity values expressed as percentage in bioluminescence with respect to control values.

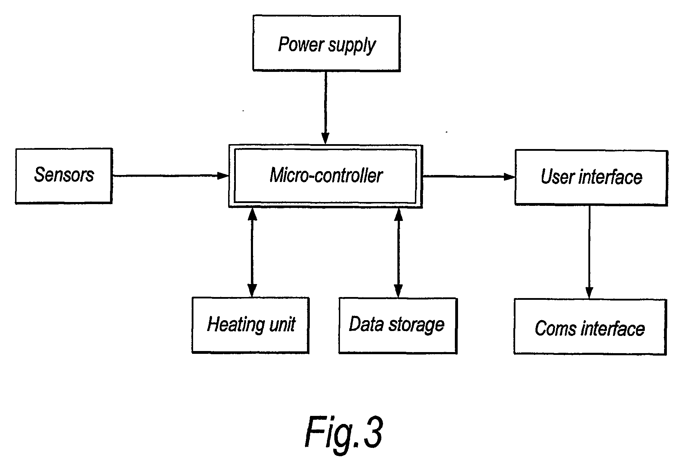

A wire or wireless port (infra-red) 53 from the micro-controller provides for downloading of data, for example stored multiple data sets, to another electronic device. Data can then be incorporated into customised software to show toxicity over time and space for the site under investigation. Figure 3 shows how the various electronic components relate to each other.

As supplied prior to use each feed tray 10 to receive soil samples contains in each of its cells 11 a specified volume of extractant liquid and is covered by a disposable sealing lid (not shown) extending over all the cells 11. A flexible plastic sheet (not shown) covers the sealing lid and together with it seals each cell 11. The sealing lid is held in place by easy-release plastic lugs 18.

In use of feed trays 10 to receive soil samples the sealing lid is removed and, after insertion of soil samples into the cells 11, is replaced by a pressurising lid 17 which is sealed to the rim of the tray 10 by a resilient plastic gasket 19 but does not seal the individual cells 11. The lid 17, which is also held in place by the plastic lugs 18, has a shaped orifice 24 to receive a pressurising syringe 25 and holds it in place with a Luer-lock fitting.

Further feed trays 10 are supplied with measured quantities of activation medium in each cell 11 to be used in activating bioluminescent bacteria.

Prior to use of the inspection tray 14, a defined quantity of freeze-dried bioluminescent bacteria is introduced into each of the cells 15. The bacteria must be activated for a defined time (typically about 60 minutes) prior to addition of solids or liquids extracts, e.g. soil extracts.

Activation is achieved by use of a further feed tray 10, pre-filled with a prescribed volume of activation medium and sealed with a disposable sealing lid and flexible plastic sheet. This activation tray 10 is first placed over the inspection tray 14, the sealing lid and sheet are removed and replaced by the pressurising lid 17. The syringe 25 is then employed to introduce air to pressurise the head space over the cells 11 and force activation medium through the porous bases to each cell 15 in the inspection tray 14.

The plastic sealing sheet is then put back onto the inspection tray 14 and the well-sealing lid clicked into place. The contents of the tray 14 are agitated for up to about 2 minutes. The plastic sheet is then removed and the well-sealing lid repositioned producing a less tight seal to allow gas exchange for the cells 15 to become metabolically active.

The tray 14 is then placed into the incubation zone 41 within the opto-electronic unit until the bacteria have reached a sufficient level of bioluminescent activity, typically after about 60 minutes. After this time the tray 14 is withdrawn from the incubation zone 41.

While incubation of the bacteria is in progress the user obtains a soil sample, for example by filling a hand-operated soil homogeniser with fresh soil, crushing the soil through a coarse filter into a plastic beaker, and using a spatula to transfer a specific volume of crushed soil into a cell 11 in a feed tray 10. A specific volume of extractant liquid is also introduced into the cell 11.

The first row of six cells 15 in the inspection tray 14 may comprise the following controls and standards (supplied with the tray): a cell with no soil extract; a cell with a standard toxin to reduce bioluminescence to zero; and four cells with standard toxins corresponding to pollutant trigger levels relevant to the site under investigation and the risk assessment system followed.

When all cells 11 contain either soil samples and extractant liquid or relevant control solutions a cell-sealing lid is placed back on the feed tray 10 which is then agitated by hand for a prescribed time. The cell-sealing lid is then replaced with the pressurising lid 17 and the tray 10 is placed on top of the inspection tray 14, from which the loose-fitting lid has been removed. The head space in the tray 10 is pressurised as before by a syringe 25 to inject extracts separated from the solids or liquids samples and control solutions into each cell 15 in the inspection tray 14. Following exposure of the bioluminscent cells to the extracts of solids or liquids, a cell-sealing lid is then placed on the inspection tray 14, which is then agitated for a period of time, for example up to 2 minutes.

When agitation is complete the cell-sealing lid is replaced by a loose fitting lid and the tray 14 is docked in the measurement zone 31 of the opto-electronic unit. The opto-electronic unit then simultaneously measures and records the level of bioluminescence emitted from each cell 15 over a specified time, for example 15 minutes. The levels are displayed on the LCD screen 52 as a two dimensional representation of the tray 14 with toxicity values expressed as percentage in bioluminescence with respect to the control values.

## Claims

1. A device for measuring the generic toxicity of solids or liquids which comprises a container to hold bioluminscent bacteria and to receive pollutants separated from the solids or liquids, and an opto-electronic unit to detect the level of bioluminscence from the container, **characterised in that** the container for bacteria and pollutants comprises one or more modular inspection trays each containing an array of cells which each have a transparent or translucent base, and the opto-electronic unit includes an array of light sensors arranged in a configuration complementary to that of the inspection trays.

2. A device as claimed in claim 1 which **characterised in that** it comprises extraction means to separate pollutants from the solids or liquids comprising one or more modular feed trays each containing an array of cells which each have a porous base, the container for bacteria and pollutants comprises one or more modular inspection trays each containing an array of cells which each have a transparent or translucent base and which are arranged in a configuration complementary to that of the feed trays, and the opto-electronic unit includes an array of light sensors arranged in a configuration complementary to those of the feed and inspection trays.

3. A device as claimed in claim 2, in which the feed trays have shaped peripheral lower edges and the inspection trays have shaped peripheral upper edges of complementary tongue and groove configurations so that when the trays are brought together for transfer of material from the upper (feed) tray to the lower (inspection) tray a seal is formed between the respective peripheries.

4. A device as claimed in claim 3, in which the tongue and groove configurations are asymmetrical so that the trays can only be fitted together in one direction.

5. A device as claimed in any preceding claims 2 to 4, in which the feed and inspection trays are constructed of moulded plastic material.

6. A device as claimed in any preceding claim 2 to 5, in which the base of each of the cells in the feed trays is constructed of porous plastic.

7. A device as claimed in any preceding claim, in which the base of each of the cells in the inspection trays is constructed of optical-grade transparent plastic.

8. A device as claimed in any preceding claim 2 to 7, in which the feed trays and the inspection trays include sealable but detachable lids.

9. A device as claimed in claim 8, in which the lids employed for the feed trays include a shaped orifice to receive a pressurising syringe to create an excess pressure in the feed trays.

10. A device as claimed in any preceding claim, in which the opto-electrical unit includes a measurement zone to receive an inspection tray containing bacteria and solids or liquids extracts.

11. A device as claimed in claim 10, in which the opto-electrical unit further includes an incubation zone to store one or more inspection trays for activation of the bacteria prior to addition of soil extracts.

12. A device as claimed in claim 11, in which the incubation zone includes a thermo control element to ensure the bacteria are incubated at optimum temperature.

13. A device as claimed in any preceding claim, in which the opto-electrical unit is battery powered.

14. A device as claimed in claim 13, in which the battery is of a rechargeable type.

15. A device as claimed in any preceding claim, in which the light sensors are photodiodes.

16. A device as claimed in claim 10, in which the light sensors are located at the base of the measurement zone, with at least one sensor per inspection cell.

17. A device as claimed in any preceding claim, in which each of the light sensors has an associated lens to focus the bioluminescence from each of the cells.

18. A device as claimed in any preceding claim, in which the opto-electrical unit includes a micro-controller to mediate data acquisition and storage.

19. A method for measuring the generic toxicity of solids or liquids samples in which pollutants are first separated from the solids or liquids, are then brought into contact with bioluminscent bacteria and the level of bioluminescence is then detected and monitored, **characterised in that** the pollutants are passed into a modular inspection tray containing an array of cells containing bioluminescent bacteria which each have a transparent or translucent base, and the level of luminscence from the inspection cells is detected by an array of light sensors arranged in a configuration complementary to that of the inspection tray.

20. A method as claimed in claim 19 **characterised in that** the soil samples are placed together with a pollutant-extracting liquid into one or more modular feed trays each containing an array of cells which each have a porous base, extracted pollutants pass through the porous bases into a modular inspection tray containing an array of cells containing bioluminescent bacteria which each have a transparent or translucent base and which are arranged in a configuration complementary to that of the feed trays, and the level of luminescence from the inspection cells is detected by an array of light sensors arranged in a configuration complementary to those of the feed and inspection trays.

21. A method as claimed in claim 19 or 20, in which the bioluminescent bacteria are selected from *Vibrio fischeri, Vibrio harveyi, Photorhabdus luminescens, Photobacterium phosphoreum,* or any genetically engineered bacterium or micro-organism or cell(s) which displays luminescence.

22. A method as claimed in claim 20 or claim 21, in which the bacteria are stored in freeze-dried form, and activated when required, by addition of an activation medium.

23. A method as claimed in claim 21, in which the activation medium includes a substance that increases the sensitivity of the bacteria a given pollutant or group or mixture thereof.

24. A method as claimed in claim 22 or claim 23, in which the activation medium includes sodium chloride (1-4% w/v pH (5-8) buffer).

25. A method as claimed in one of claims 22 to 24, in which the activation is effected by incubation at a temperature in the range 15 to 37°C.

26. A method as claimed in claim 25, in which the incubation is conducted for a period up to 120 minutes.

27. A method as claimed in any of claims 19 to 25, in which the solids or liquids samples for analysis are crushed to a particle size in the range 10 to 500 µm.

28. A method as claimed in any of claims 19 to 27, in which the extractant liquid is selected from solvents, surfactants, solutions based on ethylenediaminetetraacetic acid (EDTA) or other salts, and mixtures thereof.

29. A method as claimed in any of claims 19 to 28, in which for control purposes some of the cells in the inspection trays may comprise one or more of the following standard contents:
(a) no solids or liquids extract providing 100% bioluminescence;
(b) a standard toxin to reduce bioluminescence by 100%;
(c) one or more standard toxins corresponding to pollutant trigger levels relevant to the site under investigation and the risk assessment system followed significantly altering bioluminescence.

30. A method as claimed in claim 27, in which the standard toxin corresponding to a pollutant trigger level is selected from one or more of benzene, toluene, ethylbenzene, xylene, polyaromatic hydrocarbons, polychlorinated biphenols and heavy metals such as copper, lead, mercury or cadmium or other environmental pollutant or mixture thereof.

## Patentansprüche

1. Vorrichtung zum Messen der generischen Toxizität von Feststoffen oder Flüssigkeiten, die einen Behälter zur Aufbewahrung biolumineszenter Bakterien und zur Aufnahme von den Feststoffen oder Flüssigkeiten abgeschiedener Schadstoffe aufweist, sowie eine opto-elektronische Einheit zum Erkennen des Biolumineszenzniveaus von dem Behälter,
**dadurch gekennzeichnet, dass**
der Behälter für Bakterien und Schadstoffe einen oder mehrere modulare Kontrolleinsätze (14) aufweist, die jeweils eine Anordnung von Zellen (15) aufweisen, die jeweils einen durchsichtigen oder lichtdurchlässigen Boden (16) aufweisen, wobei die opto-elektronische Einheit eine Anordnung von Lichtsensoren (36) enthält, die in einer Konfiguration angeordnet sind, die zu demjenigen der Kontrolleinsätze (14)komplementär sind..

2. Vorrichtung nach Anspruch 1,
**dadurch gekennzeichnet, dass**
sie Extraktionseinrichtungen zur Abscheidung von Schadstoffen von den Feststoffen oder Flüssigkeiten aufweist, die einen oder mehrere modulare Zufuhreinsätze (10) aufweist, die jeweils eine Anordnung von Zellen (11) aufweisen, die jeweils einen durchsichtigen oder lichtdurchlässigen Boden (12) aufweisen, wobei der Behälter für Bakterien und Schadstoffe einen oder mehrere modulare Kontrolleinsätze (14) aufweist, die jeweils eine Anordnung von Zellen (15) aufweisen, die jeweils einen durchsichtigen oder lichtdurchlässigen Boden (16) aufweisen, die in einer Konfiguration angeordnet sind, die zu denjenigen der Zufuhreinsätze (10) komplementär sind, wobei die opto-elektronische Einheit eine Anordnung von Lichtsensoren (36) enthält, die in einer Konfiguration angeordnet sind, die zu denjenigen der Zufuhr- und Kontrolleinsätze komplementär sind.

3. Vorrichtung nach Anspruch 2,
**dadurch gekennzeichnet, dass**
die Zufuhreinsätze (10) geformte untere Umfangskanten aufweisen, und die Kontrolleinsätze (14) geformte obere Umfangskanten mit komplementären Zungen- und Nutkonfigurationen aufweisen, so dass dann, wenn die Einsätze zur Übertragung von Material von dem oberen (Zufuhr)-Einsatz (10) zu dem unteren (Kontroll)-Einsatz (14) zusammengebracht werden, eine Dichtung zwischen den jeweiligen Umfängen ausgebildet wird.

4. Vorrichtung nach Anspruch 3,
**dadurch gekennzeichnet, dass**
die Zungen- und Nutkonfigurationen asymmetrisch sind, so dass die Einsätze nur in einer Richtung aneinander montierbar sind.

5. Vorrichtung nach einem der vorangegangenen Ansprüche 2 bis 4,
**dadurch gekennzeichnet, dass**
die Zufuhr- und Kontrolleinsätze aus einem gegossenen Kunststoff hergestellt sind.

6. Vorrichtung nach einem der vorangegangenen Ansprüche 2 bis 5,
**dadurch gekennzeichnet, dass**
der Boden (12) jeder Zelle (11) in den Zufuhreinsätzen (10) aus feinporigem Kunststoff hergestellt ist.

7. Vorrichtung nach einem der vorangegangenen Ansprüche,
**dadurch gekennzeichnet, dass**
der Boden (12) jeder Zelle (11) in den Kontrolleinsätzen (14) aus durchsichtigem Kunststoff mit Optikqualität hergestellt ist.

8. Vorrichtung nach einem der vorangegangenen Ansprüche 2 bis 7,
**dadurch gekennzeichnet, dass**
die Zufuhreinsätze (10) und die Kontrolleinsätze (14) abdichtbare, jedoch abnehmbare Deckel (17) aufweisen.

9. Vorrichtung nach Anspruch 8,
**dadurch gekennzeichnet, dass**
die für die Zufuhreinsätze (10) verwendeten Deckel (17) eine geformte Öffnung aufweisen, um eine Druckbeaufschlagungsspritze (25) aufzunehmen, um einen Überdruck in den Zufuhreinsätzen (10) zu erzeugen.

10. Vorrichtung nach einem der vorangegangenen Ansprüche,
**dadurch gekennzeichnet, dass**
die opto-elektronische Einheit eine Messzone (31) aufweist, um einen Bakterien- und Feststoff- oder Flüssigkeitsextrakte enthaltenden Kontrolleinsatz (14) aufzunehmen.

11. Vorrichtung nach Anspruch 10,
**dadurch gekennzeichnet, dass**
die opto-elektronische Einheit weiterhin eine Bebrütungszone (41) zur Lagerung eines oder mehrerer Kontrolleinsätze (14) zur Aktivierung der Bakterien vor der Zugabe von Schmutzextrakten aufweist.

12. Vorrichtung nach Anspruch 11,
**dadurch gekennzeichnet, dass**
die Bebrütungszone (41) ein Thermosteuerungselement aufweist um sicherzustellen, dass die Bakterien bei optimaler Temperatur bebrütet werden.

13. Vorrichtung nach einem der vorangegangenen Ansprüche,
**dadurch gekennzeichnet, dass**
die opto-elektronische Einheit batteriebetrieben ist.

14. Vorrichtung nach Anspruch 13,
**dadurch gekennzeichnet, dass**
die Batterie wiederaufladbar ist.

15. Vorrichtung nach einem der vorangegangenen Ansprüche,
**dadurch gekennzeichnet, dass**
die Lichtsensoren (36) Fotodioden sind.

16. Vorrichtung nach Anspruch 10,
**dadurch gekennzeichnet, dass**
die Lichtsensoren (36) an dem Boden der Messzone (31) mit mindestens einem Sensor pro Kontrollzelle angeordnet sind.

17. Vorrichtung nach einem der vorangegangenen Ansprüche,
**dadurch gekennzeichnet, dass**
jeder der Lichtsensoren (36) eine dazugehörige Linse (37) besitzt, um die Biolumineszenz von jeder der Zellen zu fokussieren.

18. Vorrichtung nach einem der vorangegangenen Ansprüche,
**dadurch gekennzeichnet, dass**
die opto-elektronische Einheit einen Mikro-Controller zur Vermittlung von Datenerfassung und -speicherung aufweist.

19. Verfahren zum Messen der generischen Toxizität von Feststoff- oder Flüssigkeitsmustern, wobei Schadstoffe erst von den Feststoffen oder Flüssigkeiten abgeschieden, und dann mit biolumineszenten Bakterien in Kontakt gebracht werden, wobei dann das Biolumineszenzniveau erkannt und überwacht wird,
**dadurch gekennzeichnet, dass**
die Schadstoffe in einen modularen Kontrolleinsatz (14) weitergegeben werden, der eine Anordnung von Zellen (15) aufweist, die biolumineszente Bakterien enthalten, und die jeweils einen durchsichtigen oder lichtdurchlässigen Boden (16) aufweisen, wobei das Lumineszenzniveau von den Kontrolleinsätzen (14) durch eine Anordnung von Lichtsensoren (36) erkannt wird, die in eizu denjeniden diejenige des Kontrolleinsatzes (14) komplementär sind.

20. Verfahren nach Anspruch 19,
**dadurch gekennzeichnet, dass**
die Schmutzmuster mit einer Schadstoffextraktionsflüssigkeit gemeinsam in einem oder mehreren modularen Zufuhreinsätzen (10) positioniert werden, die jeweils eine Anordnung von Zellen (11) aufweisen, die jeweils einen feinporigen Boden (12) aufweisen, wobei extrahierte Schadstoffe durch den feinporigen Boden (12) in einen Kontrolleinsatz (14) hindurchgehen, der eine Anordnung von Zellen (15) aufweist, die biolumineszente Bakterien enthalten, und die jeweils einen durchsichtigen oder lichtdurchlässigen Boden (16) aufweisen, die in einer Konfiguration angeordnet sind, die zu denjenigen der Zufuhreinsätze (10) komplementär sind,
wobei das Lumineszenzniveau von den Kontrollzellen durch eine Anordnung von Lichtsensoren (36) erkannt wird, die in einer Konfiguration angeordnet sind, die zu denjenigen der Zufuhr- und Kontrolleinsätze komplementär sind.

21. Verfahren nach Anspruch 19 oder Anspruch 20,
**dadurch gekennzeichnet, dass**
wobei die biolumineszenten Bakterien aus Vibrio fischeri, Vibrio harveyi, Photorhabdus luminescens, Photobacterium phosphoreum, oder jedem beliebigen genetisch manipuliertem Bakterium oder Mikroorganismus oder Zelle(n) ausgewählt sind, die Lumineszenz an den Tag legen.

22. Verfahren nach Anspruch 20 oder Anspruch 21,
**dadurch gekennzeichnet, dass**
die Bakterien in gefriergetrockneter Form gelagert und wenn erforderlich durch die Zugabe eines Aktivierungsmittels aktiviert werden.

23. Verfahren nach Anspruch 21,
**dadurch gekennzeichnet, dass**
das Aktivierungsmittel eine Substanz enthält, welche die Empfindlichkeit der Bakterien gegenüber einem gegebenen Schadstoff oder Mischung davon erhöht.

24. Verfahren nach Anspruch 22 oder Anspruch 23,
**dadurch gekennzeichnet, dass**
das Aktivierungsmittel Natriumchlorid (1-4 Volumengewichts-%, pH-Wert (5-8) Puffer) enthält.

25. Verfahren nach einem der Ansprüche 22 bis 24,
**dadurch gekennzeichnet, dass**
die Aktivierung durch eine Bebrütung bei einer Temperatur in dem Bereich von 15 bis 37°C bewirkt wird.

26. Verfahren nach Anspruch 25,
**dadurch gekennzeichnet, dass**
die Bebrütung für eine Zeitdauer bis zu 120 Minuten durchgeführt wird.

27. Verfahren nach einem der Ansprüche 19 bis 25,
**dadurch gekennzeichnet, dass**
die Feststoff- oder Flüssigkeitsmuster für die Analyse auf eine Partikelgröße in dem Bereich von 10 bis 500 µm zerkleinert werden.

28. Verfahren nach einem der Ansprüche 19 bis 27,
**dadurch gekennzeichnet, dass**
die Extraktionsflüssigkeit aus Lösungsmitteln, grenzflächenaktiven Stoffen, auf Ethylendiamintetraessigsäure (EDTA) basierenden Lösungen oder anderen Salzen und Mischungen davon ausgewählt wird.

29. Verfahren nach einem der Ansprüche 19 bis 28,
**dadurch gekennzeichnet, dass**
zu Kontrollzwecken einige der Zellen (15) in den Kontrolleinsätzen (14) einen oder mehrere der folgenden Standardinhalte aufweisen können:
(a) keinen Feststoff- oder Flüssigkeitsextrakt, der 100% Biolumineszenz bereitstellt;
(b) ein Standardtoxin zur Verringerung der Biolumineszenz um 100%;
(c) eines oder mehrere Standardtoxine entsprechend den Schadstoffauslöserniveaus, die für den Untersuchungsort und das verfolgte Risikoeinschätzungssystem relevant sind, wodurch sich die Biolumineszenz bedeutend verändert.

30. Verfahren nach Anspruch 27,
**dadurch gekennzeichnet, dass**
das Standardtoxin, welches einem Schadstoffauslöserniveau entspricht, von einem oder mehreren von Benzol, Toluol, Ethylbenzol, Xylol, polyaromatischen Kohlenwasserstoffen, polychlorierten Biphenolen und Schwermetallen wie zum Beispiel Kupfer, Blei, Quecksilber oder Kadmium oder anderen Umweltschadstoffen oder Mischungen davon ausgewählt wird.

## Revendications

1. Un dispositif pour mesurer la toxicité générique de solides ou de liquides qui comprend un container pour contenir des bactéries bioluminescentes et pour recevoir des polluants isolés des solides ou des liquides, et une unité optoélectronique pour détecter le niveau de bioluminescence du container, **caractérisé en ce que** le container pour bactéries et polluants comprend un ou plusieurs plateaux modulaires d'inspection contenant chacun un ensemble de cellules ayant chacune une base transparente ou translucide, et l'unité optoélectronique comprend un ensemble de capteurs de lumière disposés dans une configuration complémentaire de celle des plateaux d'inspection.

2. Un dispositif selon la revendication 1 **caractérisé en ce qu'**il comprend des moyens d'extraction pour séparer les polluants des solides ou des liquides, lesdits moyens d'extraction comprenant un ou plusieurs plateaux modulaires d'alimentation comprenant chacun un ensemble de cellules qui ont une base poreuse, **en ce que** le container pour bactéries et polluants comprend un ou plusieurs plateaux modulaires d'inspection comprenant chacun un ensemble de cellules qui ont chacune une base transparente ou translucide et qui sont disposées dans une configuration complémentaire de celle des plateaux d'alimentation, et **en ce que** l'unité optoélectronique comprend un ensemble de capteurs de lumière disposés dans une configuration complémentaire de celle des plateaux d'alimentation et d'inspection.

3. Un dispositif selon la revendication 2, dans lequel les plateaux d'alimentation ont des bords périphériques inférieurs et les plateaux d'inspection ont des bords périphériques supérieurs mis en forme dans des configurations complémentaires de cale et rainure de cale de manière à ce que lorsque les plateaux sont regroupés pour le transfert de matériau à partir du plateau supérieur (d'alimentation) vers le plateau inférieur (d'inspection) un joint est formé entre les périphéries respectives.

4. Un dispositif selon la revendication 3, dans lequel les configurations de cale et rainure de cale sont asymétriques de manière à ce que les plateaux puissent être ajustés ensemble seulement dans une direction.

5. Un dispositif selon l'une quelconque des revendications 2 à 4, dans lequel les plateaux d'inspection et d'alimentation sont fabriqués dans une matière plastique moulée.

6. Un dispositif selon l'une quelconque des revendications 2 à 5, dans lequel la base de chacune des cellules des plateaux d'alimentation est fabriquée dans un plastique poreux.

7. Un dispositif selon l'une quelconque des revendications précédentes, dans lequel la base de chaque cellule des plateaux d'inspection est fabriquée dans un plastique transparent à qualité optique.

8. Un dispositif selon l'une quelconque des revendications 2 à 7, dans lequel les plateaux d'alimentation et d'inspection comprennent des couvercles scellables mais détachables.

9. Un dispositif selon la revendication 8, dans lequel les couvercles employés pour les plateaux d'alimentation comprennent un orifice formé pour recevoir une seringue de pressurisation pour créer un excès de pression dans les plateaux d'alimentation.

10. Un dispositif selon l'une quelconque des revendications précédentes, dans lequel l'unité optoélectrique comprend une zone de mesure pour recevoir un plateau d'inspection contenant des bactéries et des extraits de solides ou de liquides.

11. Un dispositif selon la revendication 10, dans lequel l'unité opto-électrique comprend également une zone d'incubation pour stocker un ou plusieurs plateaux d'inspection pour l'activation des bactéries préalablement à l'addition d'extraits de sol.

12. Un dispositif selon la revendication 11, dans lequel la zone d'incubation comprend un élément de contrôle thermique pour s'assurer que les bactéries soient incubées à une température optimale.

13. Un dispositif selon l'une quelconque des revendications précédentes, dans lequel l'unité opto-électrique est alimentée par une batterie.

14. Un dispositif selon la revendication 13, dans lequel la batterie est une batterie du type rechargeable.

15. Un dispositif selon l'une quelconque des revendications précédentes, dans lequel les capteurs de lumières sont des photodiodes.

16. Un dispositif selon la revendication 10, dans lequel les capteurs de lumière sont situés à la base de la zone de mesure, avec au moins un capteur par cellule d'inspection.

17. Un dispositif selon l'une quelconque des revendications précédentes, dans lequel chacun des capteurs de lumière a une lentille associée pour focaliser la bioluminescence de chacune des cellules.

18. Un dispositif selon l'une quelconque des revendications précédentes, dans lequel l'unité opto-électrique comprend un microcontrôleur pour gérer l'acquisition et le stockage de données.

19. Une méthode pour mesurer la toxicité générique d'échantillons de solides ou de liquides dans lequel des polluants sont dans un premier temps séparés des solides ou des liquides, puis sont amenés en contact avec des bactéries bioluminescentes et le niveau de bioluminescence est ensuite détecté et surveillé, **caractérisé en ce que** les polluants sont passés dans un plateau modulaire d'inspection contenant un ensemble de cellules contenant des bactéries bioluminescentes qui ont chacune une base transparente ou translucide, et le niveau de luminescence à partir de l'inspection des cellules est détecté par un ensemble de capteurs de lumière disposés dans une configuration complémentaire de celle des plateaux d'inspection.

20. Une méthode selon la revendication 19, **caractérisée en ce que** les échantillons de sol sont placés ensemble avec un liquide extracteur de polluant dans un ou plusieurs plateaux modulaires d'alimentation contenant un ensemble de cellules qui ont chacune une base poreuse, les polluants extraits passent au travers de la base poreuse vers l'intérieur d'un plateau d'inspection modulaire contenant un ensemble de cellule contenant des bactéries bioluminescentes qui ont chacune une base transparente ou translucide et qui sont disposées dans une configuration complémentaire de celle des plateaux d'alimentation, et le niveau de luminescence à partir de l'inspection des cellules est détecté par un ensemble de capteurs de lumière disposé dans une configuration complémentaire de celle des plateaux d'alimentation et d'inspection.

21. Une méthode selon la revendication 19 ou 20, dans laquelle les bactéries bioluminescentes sont sélectionnées à partir de *Vibrio fischeri, Vibrio harveyi, Photorhabdus luminescens, Photobacterium phosphoreum,* ou n'importe quelle bactérie ou cellule(s) ou micro-organisme luminescent conçu génétiquement.

22. Une méthode selon la revendication 20 ou 21, dans laquelle les bactéries sont stockées dans une forme lyophilisée, et activées le cas échéant, par addition d'un moyen d'activation.

23. Une méthode selon la revendication 21, dans laquelle le moyen d'activation comprend une substance qui accroît la sensibilité des bactéries à un polluant donné ou un groupe ou une mixture de polluants donné.

24. Une méthode selon la revendication 22 ou 23, dans laquelle le moyen d'activation comprend du chlorure de sodium (1-4% poids/volume pH (5-8) tampon).

25. Une méthode selon l'une quelconque des revendications 22 à 24, dans laquelle l'activation est effectuée par incubation à une température comprise entre 15°C et 37°C.

26. Une méthode selon la revendication 25, dans laquelle l'incubation est conduite pour une période allant jusqu'à 120 minutes.

27. Une méthode selon l'une quelconque des revendications 19 à 25, dans laquelle les échantillons de liquides ou de solides à analyser sont broyés en particules de taille de l'ordre de 10 à 500 µm.

28. Une méthode selon l'une quelconque des revendications 19 à 27, dans laquelle le liquide extracteur est sélectionné parmi les solvants, agents tensioactifs, solutions à base d'acide éthylène diamine tétra acétique et autres sels, et leurs mélanges.

29. Une méthode selon l'une quelconque des revendications 19 à 28, dans laquelle pour des raisons de contrôle une partie des cellules des plateaux d'inspection peuvent comprendre une ou plusieurs des matières standards suivantes :
(a) aucun extrait de solides ou de liquides fournissant 100% de bioluminescence
(b) une toxine standard pour réduire la bioluminescence de 100%
(c) une ou plusieurs toxines standards correspondant aux niveaux de déclenchement de polluant qui sont pertinents pour le site sous investigation et le système d'estimation du risque par suivie de l'altération significative de la bioluminescence.

30. Une méthode selon la revendication 27, dans laquelle la toxine standard correspondant à un niveau de déclenchement du polluant est sélectionnée parmi un ou plusieurs des composés suivants : benzène, toluène, éthylbenzène, xylène, hydrocarbonés poly-aromatique, biphénols poly-chlorés et métaux lourds tels que cuivre, plomb, mercure, cadmium ou autres polluants environnementaux et leurs mélanges.
